(19) Europäisches Patentamt
European Patent Office
Office européen des brevets

(11) Publication number: **0 132 194**
**B1**

(12) # EUROPEAN PATENT SPECIFICATION

(45) Date of publication of patent specification: **14.12.88**

(51) Int. Cl.$^4$: **C 07 C 126/02**

(21) Application number: **84401454.8**

(22) Date of filing: **10.07.84**

(54) **Low steam consumption process for the manufacture of urea.**

(30) Priority: **14.07.83 IT 2207383**

(43) Date of publication of application:
**23.01.85 Bulletin 85/04**

(45) Publication of the grant of the patent:
**14.12.88 Bulletin 88/50**

(84) Designated Contracting States:
**BE DE FR GB IT NL**

(56) References cited:
**EP-A-0 098 396**
**US-A-3 356 723**
**US-A-3 954 861**
**US-A-4 208 347**

**HYDROCARBON PROCESSING, November 1982, pages 87-91; G. PAGANI: "New process gives urea with less energy"**

(73) Proprietor: **Montedison S.p.A.**
**31, Foro Buonaparte**
**I-20121 Milan (IT)**

(72) Inventor: **Pagani, Giorgio**
**26/6 Viale Faenza**
**Milan (IT)**

(74) Representative: **Hirsch, Marc-Roger**
**Cabinet Hirsch 34 rue de Bassano**
**F-75008 Paris (FR)**

**EP 0 132 194 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention concerns a process having a low steam consumption for the manufacture of urea.

Improvements to urea manufacturing plants have allowed, until now, to lower the steam consumption from 1500 to 1000 kg per ton of urea or, in the most efficient case, 550—600 kg/t; U.S. patent 4,208,347 deals with a new, double loop process (I.D.R. process), but describes only the high pressure loop and does not take into account the final, low pressure, unit (the so called "finishing").

The Applicant (see: "New process gives urea with less energy" (Hydrocarbon Processing, No. 1982)) has later described an I.D.R. complete manufacturing line, "finishing" included, comprising a high pressure loop (reactor, two subsequent isobaric strippers and a carbamate condenser) and a "finishing" comprising:

I — a medium pressure still (20—30 ata), the vapors of which are condensed within a vacuum concentrator, substantially at 0.35 ata (see item II), thus realizing a multiple-effect evaporation, and a low pressure still, working substantially at 5 ata (atmospheres, absolute);

II — a two-step vacuum concentration section (respectively — and approximately — at 0.35 and 0.05 ata) and one unit for the recovery of $NH_3$ from the reaction waters and from the scrubber waters coming from the washing of the synthesis vent.

The steam consumption of the complete I.D.R. line is slightly lower than 600 kg/t and the Applicant has foreseen a further decrease to 500 kg/t.

The present invention concerns an improved process for the manufacture of urea, which process allows to lower considerably, in a new and original way, the steam consumption of said I.D.R. line; i.e. the high pressure loop and the finishing unit of the present invention were matched in an original way, so as to improve synergistically (and therefore surprisingly) the whole I.D.R. line, while lowering the (outside) steam requirement to unexpected levels.

In its most general form (see figure 1) the invention concerns an I.D.R. process for the manufacture of urea, comprising two subsequent loops, wherein:

A — in the first (high pressure) loop the solution coming from the synthesis zone contains, after the first isobaric stripper of said I.D.R. process (E1), a strong $NH_3$ excess ($NH_3$:$CO_2 \geqq 6$ by moles) and a low $CO_2$ percentage ($CO_2$:urea $\leqq$ 25% b.w.) and contains, before the second isobaric stripper (E2) of said I.D.R. process, an amount of ammonium carbamate (CBE) lower than the amount of carbmate (CBU) contained in the solution leaving the same (E2) stripper, wherein the consequent overproduction of carbamate (in E2) releases an amount of heat sufficient to displace a major proportion of the residual $NH_3$ excess contained therein, which allows a reduction of the steam wasted in (E2);

B — in the second (low pressure) loop, the solution leaving (E2) is flashed at 20—30 ata and enters a first (medium pressure) still (E3), heated indirectly and completely by low pressure recovery steam, coming from the isobaric condensation (in E8) of the vapors leaving the top of (E2), said recovery steam having such a thermal level to decompose at least said overproduction of carbamate, originated in (E2);

C — the solution leaving (E3) is flashed substantially at 5 ata and enters a second (low pressure) still (E4) and then a vacuum concentrator (E5), wherein the heat of the vapors leaving the head of (E3), rich in $NH_3$ and $CO_2$, is exploited for the working run of (E4) and (E5).

DETAILS

— The pressure of the "high pressure" loop is usually comprised from 120 to 250 kg/cm$^2$.

— The temperature of the solution leaving the first isobaric stripper (E1) is normally from 150 to 230°C.

— Said $NH_3$:$CO_2$ molar ratio of the solution leaving the first isobaric stripper E1 ($\geqq$6 by moles) concerns the total amounts of $NH_3$ and $CO_2$, not only chemically bound (e.g. as ammonium carbamate), but also for the amounts bound in the form of urea.

— Said second isobaric stripper (E2) (see European patent application 83/105592.6) is a falling-film tube-bundle heat exchanger, where the solution to be stripped meets a countercurrent stream of $CO_2$, injected from outside, the pipes being heated by condensing steam only in their uppermost portion.

— Said recovery steam (coming from E8) is at a pressure $\geqq$6.5 absolute atmospheres.

— The usual working pressure of the process side of said vacuum concentrator (E5) is approximately 0.35 absolute atmospheres.

Because of the high yields of the I.D.R. process, the ratio between the fresh $CO_2$, fed to the second isobaric stripper ($CO_2$ inj.), and the carbamate (CBE) entering the same stripper attains a very high level; in general:

$$(CO_2 \text{ inj.}):(CBE) > 2 \text{ (by moles)}$$

According to a preferred embodiment of the invention, the solution leaving (E5) must be further concentrated in (E6) (as an optional and advantageous way of carrying out the invention) exploiting the heat of the recovery steam herein-above and the resulting concentrated solution (urea = 90—95% b.w.) is transferred to the final vacuum concentrator (E7); the vapors leaving the vacuum concentrators (E5, E6 and E7), containing a certain amount of $NH_3$ and $CO_2$, are then condensed in a usual way and a recovery tower

(C3) allows to obtain a recovery stream to be condensed (in E11), together with the vapors coming from said still (E4). The resulting condensate is recycled to said (E4) still (shell side); the condensed vapors leaving (E5) are recompressed (by pump PC1) and recycled to the high pressure loop.

The first isobaric stripper (E1) of the I.D.R. process can be advantageously a vertical heat exchanger having a tube-bundle and being selected from the group comprising the so called "falling-film" and "upflow" exchangers.

The supply of steam (at 18—30 ata) to said second isobaric stripper (E2) may be also lowered to zero, thereby performing a purely adiabatic stripping.

The determination of the size of said second (E2) stripper is one of the most delicate problems of the whole process; the ratio:

$$R = \frac{\text{uppermost surface of the pipes, heated by condensing steam}}{\text{remnant portion of the surface of the pipes}}$$

should be R $\leqq$1 and preferably from ½ to ¼; i.e., the uppermost surface of the tubes should be from ½ to ⅕ (preferably from ⅕ to ⅓) of the total surface.

The residence time of the urea solution, in fact, in said uppermost portion of the exchanger must be very short, i.e. equal to or lower than 3 s (preferably 2.5 and even better from 1.5 to 2.5 s), the ratio:

$$\frac{\text{residence time in the lowermost portion}}{\text{residence time in the uppermost portion}}$$

thus being $\geqq$1 (pref. $\geqq$2, even better $\geqq$4); furthermore, the ratio between the exchanging surface ($S_{CO_2}$) of said stripper (E2) and the exchanging surface ($S_{NH_3}$) of said stripper (E1) must be preferably greater than one, namely:

$$S_{CO_2} : S_{NH_3} \geqq 1$$

According to a narrower form of the invention, it is advantageous to carry out a process in which:

a) the urea synthesis is performed at 150—250°C and 170—220 bar;
b) the temperature of the solution entering said second stripper (E2) is from 190 to 220°C;
c) the temperature of the solution leaving the bottom of the same stripper (E2) is comprised between 150 and 190°C, preferably between 160 and 190°C and advantageously between 160 and 175°C;
d) the temperature of the $CO_2$ (carbon dioxide), injected as a stripping agent into the same stripper (E2), is 80—140°C;
e) the solution leaving said first stripper (E1) and entering said second stripper (E2) contains such a low amount of $CO_2$ that:

$$CO_2 : \text{urea} \leqq 22\% \text{ b.w.};$$

f) the $NH_3$:$CO_2$ molar ratio, in said solution entering said second stripper (E2), is equal to or higher than 10.

The condensation pressure in (E5), and consequently also in (E4), of the heating vapors coming from (E3) is very low; therefore the pressure of the solution to be dstilled in (E3) can be lowered to such a level that the heating of the same still (E3) can be carried out very advantageously by means of recovery steam (at 6.5—9 ata) coming from condenser (E8).

The lowest temperature of the whole low-pressure loop (about 90°C) is registered at the inlet of the first vacuum concentrator (E5), whereafter the temperature gradually increases with the degree of concentration of the final urea solution; this is the reason why the condensation temperature on the shell side of said concentration (E5) is also very low (115—120°C) and this is why, consequently, the pressure of the whole low-pressure loop can be kept at a lower level.

The basic idea of the new process resides in the transfer of a portion of the thermal load from said second stripper (E2) to the first medium-pressure still (E3), this latter apparatus, because of the lower working pressure, being very advantageously heated by recovery steam; all this is the counterbalance of the fact that the very satisfactory stripping of ammonia in said stripper (E6) is bound to the parallel formation of carbamate, the amount of which carbamate, at the outlet of stripper (E2), is greater than at the inlet.

The improvement according to the invention is satisfactory, not only when performed within an I.D.R. process, defined in a narrow sense, but also when performed in those double-stripping processes in which the second stripper (E2) (in countercurrent with $CO_2$) is working at a pressure slightly different (several tens of atmospheres) from the pressure of the first stripper (E1); see e.g. Italian patent publication 24357 A/80.

In "a narrow sense" it is meant that the two strippers (E1) and (E2) are isobaric with each other and with the synthesis reactor, apart from the pressure drops bound to hydraulics (and similar).

As it is known, according to the equation:

$$CO_2 + 2NH_3 \rightarrow NH_2—CO—NH_2 + H_2O$$

300 kg of water are by-produced per ton of urea and such water is removed, for the most part, within two groups of vacuum concentrators, working at a different degree of vacuum.

Until now, the vapors coming from said concentrators, containing 5—7% b.w. $NH_3$ and 2—3% b.w. $CO_2$, were condensed and pumped to a recovery tower (C3 in figure 1), so as to recover the $NH_3$ contained therein in order to lower the amount of residual $NH_3$ — in the waste waters — at least to 100—200 ppm, while obtaining, from the head of the same tower C3, vapors containing 25—30% b.w. $NH_3$ (depending on the working pressure) and these vapors were condensed and recycled; the steam required by such a recovery unit was approximately 120 kg/t of urea. The new low-pressure loop of the present invention (see figure 2) is an advantageous alternative, with respect to the old kinds of process, in that said steam consumption (120 kg/t) can be completely suppressed.

According to said alternative (and to figure 2), the vapors coming from the vacuum concentrator (E6) are condensed within a water-cooled apparatus (13), into which is also conveyed a portion of the vapors (15) coming from the final vacuum concentrator (E7) (previously compressed in EJ2), the remnant portion (25) being fed, as a liquid phase, to the recovery tower (C3); the basis of such an alternative resides in that said (C3) tower contains a vertical falling-film tube-bundle, heated by the vapors (11) coming from said low-pressure still (E4), and in that the vapors (26), containing $NH_3$ and coming from said (C3) tower, are also condensed within said water-cooled apparatus (E13), whereafter the liquid condensate (27) leaving said apparatus (E13) is recycled to said tower (C3), the bottom of which tower, practically free from $NH_3$ and usually at 70—72°C, is partially wasted (line 30) and partially (line 29) utilized for the scrubbing of the synthesis vent (22).

The vapors (rich in recovered $NH_3$) leaving said apparatus (E13) (see line 31 in figure 3) are treated in a usual way; they can be precondensed in E14), compressed, for instance, by a vacuum pump PV1 (e.g. at 1—1.5 kg/cm$^2$, absolute) and then condensed (in E15), further compressed (by pump PC2), cooled in (E16), together with the scrubbing liquid (35) from tower (scrubber) (C2). The steam consumption is thus suppressed, while there is a slightly higher power need, (approximately 2 Kwh per t of urea).

The performance of said apparatus (E13) can be surprisingly improved, when using, as shown in figure 3, a vertical exchanger, containing a tube-bundle, wherein the cooling medium, e.g. $H_2O$ flows within the bundle pipes, said exchanger being characterized in that the vapors to be (at least partially) condensed enter the lowermost portion of the shell and leave the same shell in its uppermost portion, thus flowing in countercurrent to the liquid condensate, which falls downwards, at least partially along the outer surface of the pipes, and which leaves the lowermost portion of said shell, in a lower position, with respect to the inlet of the vapors. The following examples illustrate the invention without however limiting in any sense the scope thereof.

Example 1

Operative conditions and compositions of the process lines are recorded on Table 1.

According to figure 1, the effluent (3), coming from reactor R1, was transferred to the first (falling-film) stripper (E1), isobaric with said reactor, in countercurrent to a $NH_3$ stream (2). It is incidentally noted that this stripper (E1) can obviously also work in a purely thermal way, namely without any injection of $NH_3$ from outside; furthermore, the falling-film apparatus can be replaced by an "up-flow" apparatus, like the one indicated in figure 4. The solution leaving (E1) flowed into the second (falling-film) isobaric stripper (E2), in countercurrent to a $CO_2$ stream (24). The solution (6) leaving (E2) was flashed from 195 to 25 kg/cm$^2$ (absolute) and flowed to the medium pressure (falling-film) still (E3) and the solution (8) leaving the bottom of (E3) was in turn flashed to 5 kg/cm$^2$ (absolute) and transferred to the low-pressure (falling-film) still (E4). The steam condensates coming (at about 225°C) from (E1) and (E2) were used for heating part of the $NH_3$ feed (at 200°C), while the remnant portion of $NH_3$ was pre-heated (at 150—160°C) by means of recovery steam. The vapors (9) leaving the head of (E3) supplied, while condensing within the shell side, all the heat required by said still (E4) and by the first vacuum concentrator (E5), working at about 0.35 kg/cm$^2$ (absolute), where the solution was concentrated up to about 85% b.w. (of urea); a further vacuum concentrator (E6), also working at 0.35 kg/cm$^2$, and the final vacuum concentrator (E7), working at 0.05 kg/cm$^2$ (absolute), brought the concentration up to 99.7% b.w., whereafter the molten urea (13) was ready to flow to a final prilling section (P) or to other treatment or use, of quite current nature.

The vapors (14) and (15) leaving the head of the vacuum concentrators (E6) and (E7) were condensed within a usual condensation system and were transferred, in the liquid state, to the recovery column (C3), from which a recovery vapor (20) was obtained, that was condensed in (E11), together with the head vapors (11) of the still (E4); the resulting condensate (21) entered the shell side of the same (E4) still. The condensed vapors (23) leaving (E5) were recompressed from 25 to 195 kg/cm$^2$ (absolute) and then conveyed (in part, namely about 90%) to the high pressure (isobaric) condenser (E8), by means of ejector (EJ1); the remnant (about 10%) was used as a scrubbing liquid for the vent coming from the reactor (tower C1 in figure 1).

The steam balance is recorded on table A; as the urea output was 43,863 kg/h, the medium-pressure steam waste (at 25 kg/cm$^2$) was: (13,158:43,863) × 1000 = 300 kg/t of urea and the low-pressure steam waste (at 8 kg/cm$^2$) was only: (40,120—36,845): 43,863 × 1000 = 75 kg/t.

In other words, the process was nearly self-supporting (as to the low-pressure steam requirement) and the medium-pressure steam consumption was limited to 300 kg/t, which was indeed new and surprising.

These results can be moreover improved by further decreasing the low-pressure steam consumption; in particular, a new recovery system (i.e. recovery of $NH_3$ from the reaction waters), allowed to suppress completely the low-pressure steam. The basic idea of such a new system was the evaporation of said waters at the same pressure of the first vacuum concentrator (E5), the heat being supplied by a partial condensation of the vapors coming from (E4); said new system is described in detail hereinafter in Example 2.

Example 2

Example 1 was repeated, while replacing the recovery operations of figure 1 (hatch area) by the operations shown in figure 2; data and results were recorded on Table 2 and the steam balance on Table A.

Acording to said figure 2, to said Table 2 and to the preceding figure 1, said vapors (15) coming from (E6) were condensed within said apparatus (E13), and said vapors (15) coming from (E7) were condensed (for the most part) first in the water-cooled apparatus (E17) and then in (E13), after a pressure increase, by means of ejector EJ2, driven by low-pressure steam. A condensated portion (25) of said vapors (15) left the (E17) cooler and entered the recovery tower (C3), consisting of a vertical falling-film tube-bundle, heated by the vapors (11) coming from the still (E4). Also the vapors (26) coming from the (C3) tower entered said apparatus (E13), whereafter the liquid condensate (27) leaving same apparatus (E13) was recycled to said tower (C3), the bottom of which tower, practically free from $NE_3$ and about at 70°C, was partially wasted (line 30) and partially (line 29) utilized in (C2), namely in the scrubber of the vent gases (22). Said waste (30) to disposal practically corresponds to the synthesis water plus the amount of steam used for the driving of ejector EJ2. The vapors (31), rich in recovered $NH_3$ and leaving the head of (E13), were partially condensed within the (E14) cooler; the condensate (through pump PC7) and the residual vapors (through vacuum pump PV1) were transferred to a condensation system (E15) working at about 1.5 kg/cm$^2$ (absolute). The condensate herefrom was transferred, through pump (PC2), to the final condensation system (E16), working at 5 kg/cm$^2$ (absolute), to which also flowed the condensing mixture (28) coming from tower (C3), the cooling medium being the fresh $NH_3$ of the feed, which was thus pre-heated to 70°C.

The solution (34) thus obtained joined solution (35) coming from abatement scrubber (C2) and the mixture flowed to the shell side of the low-pressure still (E4). Such a recovery cycle allowed to remove all the synthesis water, while using recovery steam; the steam requirement (from outside) for the distillation (stills E3 and E4) and for the first vacuum concentrator (E5) was therefore zero, while the power requirement for the vacuum pump (PV1) was merely about 2 Kwh/t of urea. With respect to example 1, we registered the output of an excess of low-pressure steam (at 8 kg/cm$^2$, absolute), that can be calculated as follows (see Table A): (36,845—34,406):43,863 × 1000 = 55 kg/t.

Example 3

Example 2 was repeated, while modifying the first isobaric stripper (E1), which was an "up-flow" equicurrent apparatus, instead of the former falling-film (countercurrent) exchanger (see figure 4); no stripping gas was injected from outside, as a coadjuvant agent. Furthermore, the working of the second isobaric stripper (E2) was adiabatic; no indirect heat (namely no condensing steam) was supplied to (E2) and the displacement of the $NH_3$ excess was merely bound to the parallel formation of carbamate and to the "self-cooling" of the solution. Moreover, a portion (36) of the vapors leaving the head of said second stripper (E2) was recycled directly to the synthesis reactor (R1), because of the thermal balance in a point higher than the inlet of the vapors coming from said first stripper (E1). Within said reactor the $NH_3$:$CO_2$ molar ratio was 4.5, the $H_2O/CO_2$ molar ratio was 0.4 and the carbamate conversion per pass was 73%. Data and results are on Tables 3 and 4; the $NH_3$ recovery cycle (from the synthesis waters) is still the cycle of example 2 and the steam balance is recorded on Table A. The reduction of the medium-pressure steam consumption to 150 kg/t (see Table A) allows to approximate considerably the exciting threshold of a zero steam requirement, namely the thermal independence.

Example 4

Example 3 was repeated, apart from the differences hereinafter: more heat was supplied to the (E3) (by means of an increase of the recovery steam coming from E8), namely more than the heat required by (E4) and (E5); the consequent excess of heat was recovered by cooling the vapors (9) leaving (E3), rich in $NH_3$ and $CO_2$, within a proper exchanger (E10/bis), which pre-heated the $NH_3$ fresh feed (see figure 5).

Such a new exchanger was connected in series with (E4) and (E5) and was fed not only by vapors (9) described herein-above but also by a liquid phase (21) in order to speed up the condensation of the same vapors (9) (see figure 5). The steam balance was recorded on Table A. Should the exchanger E10/bis be connected in parallel, results would be nearly the same.

TABLE A

| AMOUNTS OF STEAM | EXAMPLE 1 | EXAMPLE 2 | EXAMPLE 3 | EXAMPLE 4 |
|---|---|---|---|---|
| Waste of medium pressure saturated steam (at 25 kg/cm², absclute): | | | | |
| - to the first isobaric stripper (E1) | 10088 kg/h | 10088 kg/h | 6580 kg/h | 6580 kg/h |
| - to the second isobaric stripper (E2) | 3070 kg/h | 3070 kg/h | - | - |
| Total | 13158 kg/h | 13158 kg/h | 6580 kg/h | 6580 kg/h |
| Waste of low-pressure saturated steam (at 8 kg/cm², absolute): | | | | |
| - to still (E3) | 17545 kg/h | 17545 kg/h | 15483 kg/h | 17676 kg/h |
| - to vacuum concentrator (E6) | 5263 kg/h | 5263 kg/h | 5263 kg/h | 5263 kg/h |
| - to vacuum concentrator (E7) | 3948 kg/h | 3948 kg/h | 3948 kg/h | 3948 kg/h |
| - to vacuum system (driving of a battery of ejectors) | 1754 kg/h | 420 kg/h | 420 kg/h | 420 kg/h |
| - to recovery tower (C3) | 4380 kg/h | - | - | - |
| - to $NH_3$ pre-heating | 4380 kg/h | 4380 kg/h | 5910 kg/h | 1530 kg/h |
| - to steam-tracings | 2850 kg/h | 2850 kg/h | 2850 kg/h | 2850 kg/h |
| Total | 40120 kg/h | 34406 kg/h | 33874 kg/h | 31687 kg/h |
| Recovery of saturated steam (at 8 kg/cm², absolute) from (E8) | 36845 kg/h | 36485 kg/h | 31669 kg/h | 31669 kg/h |
| Summary (unitary amounts) | | | | |
| Waste of 25 kg/cm² steam (kg/t of urea) | 300 kg/t | 300 kg/t | 150 kg/t | 150 kg/t |
| Recovery (balance) of 8 kg/cm² steam | -75 kg/t(*) | +55 kg/t | -50 kg/t(*) | - |

(*) Waste

TABLE 1

Page I

| LINE | 1 | | 2 | | 3 | | 4 | | 5 | | 6 | |
|---|---|---|---|---|---|---|---|---|---|---|---|---|
| State | Liquid | | Gas | | Liquid | | Vapor | | Liquid | | Liquid | |
| P ($kg/cm^2$ abs.) | | | 195 | | 195 | | 195 | | 195 | | 195 | |
| T (°C) | | | 200 | | 194 | | 195 | | 203 | | 175 | |
| COMPOSITION | kg/h | % b.w. | kg/h | % b.w. | kg/h | % b.w. | kg/h | % b.w. | kg/h | % b.w. | kg/h | % b.w. |
| Urea | - | - | - | - | 43,863 | 34.50 | - | - | 43,863 | 37.22 | 43,863 | 45.77 |
| $NH_3$ | 24,856 | 100.00 | 7,500 | 100.00 | 45,634 | 35.89 | 11,396 | 67.81 | 41,738 | 35.42 | 14,135 | 14.76 |
| $CO_2$ | - | - | - | - | 13,789 | 10.84 | 5,043 | 30.00 | 8,746 | 7.42 | 15,343 | 16.01 |
| $H_2O$ | - | - | - | - | 23,865 | 18.77 | 367 | 2.19 | 23,498 | 19.94 | 22,480 | 23.46 |
| TOTAL | 24,856 | 100.00 | 7,500 | 100.00 | 127,151 | 100.00 | 16,806 | 100.00 | 117,845 | 100.CO | 95,821 | 100.00 |

TABLE 1

Page II

| LINE | 7 | | 8 | | 9 | | 10 | | 11 | | 12 | |
|---|---|---|---|---|---|---|---|---|---|---|---|---|
| State | Liquid | | Gas | | Liquid | | Vapor | | Liquid | | Liquid | |
| P ($kg/cm^2$ abs.) | 195 | | 25 | | 25 | | 5 | | 5 | | 0,35 | |
| T (°C) | 200 | | 157 | | 160 | | 135 | | 135 | | 115 | |
| COMPOSITION | kg/h | % b.w. | kg/h | % b.w. | kg/h | % b.w. | kg/h | % b.w. | kg/h | % b.w. | kg/h | % b.w. |
| Urea | - | - | 43,863 | 60.20 | - | - | 43.863 | 72.00 | | - | 43,863 | 92.26 |
| $NH_3$ | 27,603 | 50.94 | 5,422 | 7.44 | 8,713 | 37.95 | 914 | 1.50 | 4,508 | 37.74 | 119 | 0.25 |
| $CO_2$ | 25,569 | 47.18 | 2,889 | 3.96 | 12,454 | 54.25 | 305 | 0.50 | 2,584 | 21.64 | 47 | 0.10 |
| $H_2O$ | 1,018 | 1.88 | 20,690 | 28.40 | 1,790 | 7.80 | 15,839 | 26.00 | 4,851 | 40.62 | 3,516 | 7.39 |
| TOTAL | 54,190 | 100.00 | 72,864 | 100.00 | 22,957 | 100.00 | 60,921 | 100.00 | 11,943 | 100.00 | 47,545 | 100.00 |

TABLE 1

Page III

| LINE | 13 | | 14 | | 15 | | 16 | | 17 | | 18 | |
|---|---|---|---|---|---|---|---|---|---|---|---|---|
| State | Liquid | | Vapor | | Vapor | | Liquid | | Liquid | | Liquid | |
| P (kg/cm² abs.) | 0,05 | | 0,35 | | 0,05 | | - | | - | | - | |
| T (°C) | 140 | | 120 | | 140 | | 40 | | 40 | | 40 | |
| COMPOSITION | kg/h | % b.w. | kg/h | % b.w. | kg/h | % b.w. | kg/h | % b.w. | kg/h | % b.w. | kg/h | % b.w. |
| Urea | 43,863 | 99.70 | - | - | - | - | - | - | - | - | - | - |
| $NH_3$ | - | - | 795 | 5.94 | 119 | 3.35 | 914 | 4.89 | - | - | 758 | 4.89 |
| $CO_2$ | - | - | 258 | 1.93 | 47 | 1.32 | 305 | 1.63 | - | - | 253 | 1.63 |
| $H_2O$ | 132 | 0.30 | 12,323 | 92.13 | 3,384 | 95.33 | 17,457 | 93.48 | 14,777 | 100.00 | 14,489 | 93.48 |
| TOTAL | 43,995 | 100.00 | 13,376 | 100.00 | 3,550 | 100.00 | 18,676 | 100.00 | 14,777 | 100.00 | 15,500 | 100.00 |

TABLE 1

Page IV

| LINE | 19 | | 20 | | 21 | | 22 | | 23 | | 24 | |
|---|---|---|---|---|---|---|---|---|---|---|---|---|
| State | Liquid | | Vapor | | Liquid | | Gas (*) | | Liquid | | Gas | |
| P (kg/cm² abs.) | 5 | | 5 | | 25 | | 195 | | 25 | | 195 | |
| T (°C) | 60 | | 80 | | 50 | | about 195 | | 125 | | 130 | |
| COMPOSITION | kg/h | % b.w. | kg/h | % b.w. | kg/h | % b.w. | kg/h | % b.w. | kg/h | % b.w. | kg/h | % b.w. |
| Urea | - | - | - | - | - | - | - | - | - | - | - | - |
| $NH_3$ | 304 | 8.73 | 1,062 | 25.25 | 5,570 | 34,39 | 148 | - | 14,283 | 36.52 | - | - |
| $CO_2$ | 210 | 6.03 | 463 | 11.01 | 3,047 | 18.87 | 158 | - | 15,501 | 39.64 | 32,166 | 100.00 |
| $H_2O$ | 2,968 | 85.24 | 2,680 | 63.74 | 7,531 | 46.64 | - | - | 9,321 | 23.84 | - | - |
| TOTAL | 3,482 | 100.00 | 4,205 | 100.00 | 16,148 | 100.00 | 306 | 100.00 | 39,105 | 100.00 | 32,166 | 100.00 |

* Inert gas + $NH_3$ + $CO_2$

TABLE 2

Page I

| LINE | 25 | | 26 | | 27 | | 28 | | 29 | | 30 | |
|---|---|---|---|---|---|---|---|---|---|---|---|---|
| State | Liquid | | Vapor | | Liquid | | Liquid + Vapor | | Liquid | | Liquid | |
| P ($kg/cm^2$ abs.) | 0.05 | | 0.35 | | 0.35 | | 5 | | 0.35 | | 1 | |
| T (°C) | 40 | | 60 | | 40 | | 85 | | 70 | | 70 | |
| COMPOSITION | kg/h | % b.w. | kg/h | % b.w. | kg/h | % b.w. | kg/h | % b.w. | kg/h | % b.w. | kg/h | % b.w. |
| Urea | - | - | - | - | - | - | - | - | - | - | - | - |
| $NH_3$ | 71 | 2.08 | 176 | 4.83 | 105 | 0.75 | 4,508 | 37.74 | - | - | - | - |
| $CO_2$ | 47 | 1.38 | 75 | 2.05 | 28 | 0.20 | 2,584 | 21.64 | - | - | - | - |
| $H_2O$ | 3,295 | 96.54 | 3,400 | 93.12 | 13,852 | 99.05 | 4,851 | 40.62 | 300 | 100.00 | 13,447 | 100.00 |
| TOTAL | 3,413 | 100.00 | 3,651 | 100.00 | 13,985 | 100.00 | 11,943 | 100.00 | 300 | 100.00 | 13,447 | 100.00 |

**TABLE 2**

**Page II**

| LINE | 31 | | 32 | | 33 | | 34 | | 35 | |
|---|---|---|---|---|---|---|---|---|---|---|
| State | Vapor | | Liquid | | Liquid | | Liquid | | Liquid | |
| P (kg/cm² abs.) | 0.35 | | 0.35 | | 5 | | 5 | | 25 | |
| T (°C) | 50 | | 50 | | 40 | | 50 | | 50 | |
| COMPOSITION | kg/h | % b.w. | kg/h | % b.w. | kg/h | % b.w. | kg/h | % b.w. | kg/h | % b.w. |
| Urea | - | - | - | - | - | - | - | - | - | - |
| $NH_3$ | 914 | 25.40 | 390 | 12.93 | 914 | 25.40 | 5,422 | 34.88 | 148 | 24.42 |
| $CO_2$ | 305 | 8.47 | 305 | 10.12 | 305 | 8.47 | 2,889 | 18.59 | 158 | 26.07 |
| $H_2O$ | 2,380 | 66.13 | 2,320 | 76.95 | 2,580 | 66.13 | 7,231 | 46.53 | 300 | 49.51 |
| TOTAL | 3,599 | 100.00 | 3,015 | 100.00 | 3,599 | 100.00 | 15,542 | 100.00 | 606 | 100.00 |

TABLE 3

Page I

| LINE | 1 | | 2 | | 3 | | 4 | | 5 | | 6 | |
|---|---|---|---|---|---|---|---|---|---|---|---|---|
| State | Liquid | | Gas | | Gas | | Vapor | | Liquid | | Liquid | |
| P ($kg/cm^2$ abs.) | | | 190 | | 190 | | 190 | | 190 | | 190 | |
| T (°C) | | | 200 | | 190 | | 200 | | 200 | | 165 | |
| COMPOSITION | kg/h | % b.w. | kg/h | % b.w. | kg/h | % b.w. | kg/h | % b.w. | kg/h | % b.w. | kg/h | % b.w. |
| Urea | | | | | 43,863 | 34.30 | - | - | 43,863 | 37.27 | 43,863 | 49.25 |
| $NH_3$ | 24,856 | 100.00 | 3,500 | 100.00 | 51,752 | 40.47 | 9,697 | 95.16 | 42,055 | 35.73 | 12,055 | 13.54 |
| $CO_2$ | | | | | 11,897 | 9.30 | 343 | 3.37 | 11,554 | 9.82 | 13,915 | 15.63 |
| $H_2O$ | | | | | 20,367 | 15.93 | 150 | 1.47 | 20,217 | 17.18 | 19,217 | 21.58 |
| TOTAL | 24,856 | 100.00 | 3,500 | 100.00 | 127,879 | 100.00 | 10,190 | 100.00 | 117,689 | 100.00 | 89,050 | 100.00 |

**TABLE 3**

**Page II**

| LINE | 7 | | 8 | | 9 | | 10 | | 11 | | 12 | |
|---|---|---|---|---|---|---|---|---|---|---|---|---|
| State | Vapor | | Liquid | | Vapor | | Liquid | | Vapor | | Liquid | |
| P (kg/cm² abs.) | 190 | | 25 | | 25 | | 5 | | 5 | | 0.35 | |
| T (°C) | 295 | | 155 | | 155 | | 135 | | 135 | | 115 | |
| COMPOSITION | kg/h | % b.w. | kg/h | % b.w. | kg/h | % b.w. | kg/h | % b.w. | kg/h | % b.w. | kg/h | % b.w. |
| Urea | - | - | 43,863 | 61.31 | - | - | 43,863 | 72.00 | | - | 43,863 | 92.26 |
| $NH_3$ | 28,088 | 49.34 | 4,792 | 6.70 | 7,263 | 41.48 | 914 | 1.50 | 3,878 | 36.51 | 119 | 0.25 |
| $CO_2$ | 27,906 | 49.02 | 4,515 | 6.31 | 9,400 | 53.69 | 305 | 0.50 | 4,210 | 39.64 | 47 | 0.10 |
| $H_2O$ | 936 | 1.64 | 18,372 | 25.68 | 845 | 4.83 | 15,839 | 26.00 | 2,533 | 23.85 | 3,516 | 7.39 |
| TOTAL | 56,930 | 100.00 | 71,542 | 100.00 | 17,508 | 100.00 | 60,921 | 100.00 | 10,621 | 100.00 | 47,545 | 100.00 |

TABLE 3

Page III

| LINE | 13 | | 14 | | 15 | |
|---|---|---|---|---|---|---|
| State | Liquid | | Vapor | | Vapor | |
| P (kg/cm² abs.) | 0.05 | | 0.35 | | 0.05 | |
| T (°C) | 140 | | 120 | | 140 | |
| COMPOSITION | kg/h | % b.w. | kg/h | % b.w. | kg/h | % b.w. |
| Urea | 43,863 | 99.70 | - | - | - | - |
| $NH_3$ | - | - | 795 | 5.95 | 119 | 3.35 |
| $CO_2$ | - | - | 258 | 1.93 | 47 | 1.32 |
| $H_2O$ | 132 | 0.30 | 12,323 | 92.13 | 3,384 | 95.33 |
| TOTAL | 43,995 | 100.00 | 13,376 | 100.00 | 3,550 | 100.00 |

**TABLE 3**

**Page IV**

| LINE | 21 | | 22 | | 23 | | 24 | |
|---|---|---|---|---|---|---|---|---|
| State | Liquid | | Gas (*) | | Liquid | | Gas | |
| P (kg/cm² abs.) | 25 | | 190 | | 25 | | 190 | |
| T (°C) | 70 | | circa 190 | | 125 | | 130 | |
| COMPOSITION | kg/h | % b.w. | kg/h | % b.w. | kg/h | % b.w. | kg/h | % b.w. |
| Urea | - | - | - | - | - | - | - | - |
| $NH_3$ | 4,940 | 33,42 | 148 | - | 12,203 | 37.74 | - | - |
| $CO_2$ | 4,673 | 31.52 | 158 | - | 14,203 | 43.52 | 32,166 | 100.00 |
| $H_2O$ | 5,213 | 35.16 | - | - | 6,058 | 18.74 | - | - |
| TOTAL | 14,826 | 100.00 | 306 | 100.00 | 32,334 | 100.00 | 32,166 | 100.00 |

TABLE 4

Page I

| LINE | 25 | | 26 | | 27 | | 28 | | 29 | | 30 | |
|---|---|---|---|---|---|---|---|---|---|---|---|---|
| State | Liquid | | Vapor | | Liquid | | Liquid + Vapor | | Liquid | | Liquid | |
| P (kg/cm² abs.) | 0.05 | | 0.35 | | 0.35 | | 5 | | 0.35 | | 1 | |
| T (°C) | 40 | | 60 | | 40 | | 85 | | 70 | | 70 | |
| COMPOSITION | kg/h | % b.w. | kg/h | % b.w. | kg/h | % b.w. | kg/h | % b.w. | kg/h | % b.w. | kg/h | % b.w. |
| Urea | - | - | - | - | - | - | - | - | - | - | - | - |
| $NH_3$ | 71 | 2.08 | 176 | 4.83 | 105 | 0.75 | 3,878 | 36.51 | - | - | - | - |
| $CO_2$ | 47 | 1.38 | 75 | 2.05 | 28 | 0.20 | 4,210 | 30.64 | - | - | - | - |
| $H_2O$ | 3,295 | 96.54 | 3,400 | 93.12 | 13,852 | 99.05 | 2,533 | 23.85 | 300 | 100.00 | 13,447 | 100.00 |
| TOTAL | 3,413 | 100.00 | 3,651 | 100.00 | 13,985 | 100.00 | 10,621 | 100.00 | 300 | 100.00 | 13,447 | 100.00 |

TABLE 4

Page II

| LINE | 31 | | 32 | | 33 | | 34 | | 35 | | 36 | |
|---|---|---|---|---|---|---|---|---|---|---|---|---|
| State | Vapor | | Liquid | | Liquid | | Liquid | | Liquid | | Vapor | |
| P (kg/cm² abs.) | 0.35 | | 0.35 | | 5 | | 5 | | 25 | | 190 | |
| T (°C) | 50 | | 40 | | 40 | | 50 | | 50 | | 200 | |
| COMPOSITION | kg/h | % b.w. | kg/h | % b.w. | kg/h | % b.w. | kg/h | % b.w. | kg/h | % b.w. | kg/h | % b.w. |
| Urea | - | - | - | - | - | - | - | - | - | - | - | - |
| $NH_3$ | 914 | 25.40 | 390 | 12.93 | 914 | 25.40 | 4,792 | 33.70 | 148 | 24.42 | 1,912 | 49.34 |
| $CO_2$ | 305 | 8.47 | 305 | 10.12 | 305 | 8.47 | 4,515 | 31.75 | 158 | 26.07 | 1,899 | 49.01 |
| $H_2O$ | 2,380 | 66.13 | 2,320 | 76.95 | 2,380 | 66.13 | 4,913 | 34.55 | 300 | 49.51 | 64 | 1.65 |
| TOTAL | 3,599 | 100.00 | 3,015 | 100.00 | 3,599 | 100.00 | 14,220 | 100.00 | 606 | 100.00 | 3,875 | 100.00 |

**Claims**

1. An I.D.R. process for the manufacture of urea, comprising two subsequent loops, wherein:

A — in the first (high pressure) loop the solution coming from the synthesis zone contains, after the first isobaric stripper (E1), a strong $NH_3$ excess ($NH_3:CO_2 \geq 6$ by moles) and a low $CO_2$ percentage ($CO_2$:urea $\leq$ 25% b.w.) and contains, before the second isobaric stripper (E2), an amount of ammonium carbamate lower than the amount of carbmate contained in the solution leaving the same (E2) stripper, wherein the consequent overproduction of carbamate (in E2) releases an amount of heat sufficient to displace a major proportion of the residual $NH_3$ excess contained therein;

B — in the second (low pressure) loop, the solution leaving (E2) is flashed at 20—30 ata and enters a first (medium pressure) still (E3), heated indirectly and completely by a low pressure recovery steam, coming from the isobaric condensation (in E8) of the vapors leaving the top of (E2), said recovery steam having such a thermal level to decompose at least said overproduction of carbamate, originated in (E2);

C — the solution leaving (E3) is flashed substantially at 5 ata and enters a second (low pressure) still (E4) and then a vacuum concentrator (E5), wherein the heat of the vapors leaving the head of (E3), rich in $NH_3$ and $CO_2$, is exploited for the working run of (E4) and (E5).

2. A process according to claim 1, wherein:

— the pressure of the "high pressure" loop is from 120 to 250 kg/cm$^2$;

— the temperature of the solution leaving the first isobaric stripper (E1) is from 150 to 230°C;

— said $NH_3:CO_2$ molar ratio ($\geq$6 by moles at the outlet of the first stripper) concerns the total amounts of $NH_3$ and $CO_2$, not only if chemically bound, but also for the amounts bound in the form of urea;

— said second isobaric stripper (E2) is a falling-film tube-bundle heat exchanger, where the solution to be stripped meets a countercurrent stream of $CO_2$, injected from outside, the pipes being heated by condensing steam only in their uppermost portion;

— said recovery steam (coming from E8) is at a pressure $\geq$6.5 absolute atmospheres;

— the working pressure of the process side of said vacuum concentrator (E5) is approximately 0.35 absolute atmospheres;

— the ratio between the fresh $CO_2$, fed to the second isobaric stripper ($CO_2$ inj.), and the carbamate (CBE) entering the same stripper attains a very high level, namely:

$$(CO_2 \text{ inj.}):(CBE) > 2 \text{ (by moles)}.$$

3. A process according to claim 1, wherein said first isobaric stripper (E1) of the I.D.R. process is a vertical heat exchanger having a tube-bundle and being selected from the group comprising the "falling-film" and the "upflow" exchangers.

4. A process according to claim 1, wherein the supply of steam (at 18—30 ata) to said second isobaric stripper (E2) is lowered to zero, performing thereby a purely adiabatic stripping.

5. A process according to claim 1, wherein the solution leaving (E5) is further concentrated in (E6) exploiting the heat of the recovery steam herein-above, and the resulting concentrated solution is transferred to a final vacuum concentrator (E7), wherein the vapors leaving the vacuum concentrators (E5, E6 and E7), containing some amounts of $NH_3$ and $CO_2$, are condensed in a usual way and wherein a recovery tower (C3) allows to obtain a recovery steam to be condensed (in E11), together with the vapors coming from said still (E4), the resulting condensate being recycled to said (E4) still (shell side) and the condensed vapors leaving (E5) being recompressed and recycled to the high pressure loop.

6. A process according to claim 5, wherein (within said second stripper E2) the ratio:

$$R = \frac{\text{uppermost surface of the pipes, heated by condensing steam}}{\text{remnant portion of the surface of the pipes}}$$

is R $\leq$1, and preferably from ½ to ¼, the uppermost surface of the pipes thus being from ½ to ⅕ (preferably from ⅙ to ⅓) of the total surface.

7. A process according to claim 5, wherein the residence time of the urea solution, in said uppermost portion of the exchanger (E2) is equal to or lower than 3 s (preferably 2.5 and advantageously from 1.5 to 2.5 s), the ratio:

$$\frac{\text{residence time in the lowermost portion}}{\text{residence time in the uppermost portion}}$$

thus being $\geq$1 (pref. $\geq$2, advantageously $\geq$4).

8. A process according to claim 5, wherein:

a) the urea synthesis is performed at 150—250°C and 170—220 atmospheres;

b) the temperature of the solution entering said second stripper (E2) is from 190 to 220°C;

c) the temperature of the solution leaving the bottom of the same stripper (E2) is from 150 and 190°C, preferably from 160 and 190°C and advantageously from 160 to 175°C;

d) the temperature of the $CO_2$, injected as a stripping agent into the same stripper (E2), is from 80 to 140°C;

e) the solution leaving said first stripper (E1) and entering said second stripper (E2) contains a low amount of $CO_2$ so that:

$$CO_2 : \text{urea} \leq 22\% \text{ b.w.};$$

f) the $NH_3$:$CO_2$ molar ratio, in said solution entering said second stripper (E2), is equal to or higher than 10.

9. A process according to claim 5, wherein the ratio between the exchanging surface ($S_{CO_2}$) of said stripper (E2) and the exchanging surface ($S_{NH_3}$) of said stripper (E1) is greater than one, wherein namely:

$$S_{CO_2} : S_{NH_3} > 1.$$

10. A process according to claim 5, wherein a portion of the vapors leaving the head of said stripper (E2) was recycled directly to the synthesis reactor, without flowing through high pressure condenser (E8), in a point higher than the inlet of the vapors coming from said first stripper (E1).

11. A process according to claim 5, wherein the vapors (14) coming from vacuum concentrators (E5) and (E6) are condensed within a water-cooled apparatus (E13), into which is also conveyed a portion of the vapors (15) coming from the final vacuum concentrator (37) (previously compressed in E12), the remnant portion (25) being fed, as a liquid phase, to the recovery tower (C3), wherein said (C3) tower contains a vertical falling-film tube-bundle, heated by the vapors (11) coming from said low-pressure still (E4), and wherein the vapors (26), containing $NH_3$ and coming from said (C3) tower, are also condensed within said water-cooled apparatus (E13), whereafter the liquid condensate (27) leaving said apparatus (E13) is recycled to said tower (C3), the bottom of which tower, practically free from $NH_3$, is partially wasted (line 30) and partially utilized (line 29) for the scrubbing of the synthesis vent (22).

12. A condensing apparatus, particularly suitable for the working of the apparatus (E13) of claim 11, said condensing apparatus being a vertical exchanger, containing a tube-bundle, wherein the cooling medium flows within the bundle pipes, said exchanger being characterized in that the vapors to be (at least partially) condensed enter the lowermost portion of the shell and leave the same shell in its uppermost portion, thus flowing in countercurrent to the liquid condensate, which falls downwards and which leaves the lowermost portion of said shell, in a lower position, with respect to the inlet of the vapors.

**Patentansprüche**

1. I.D.R.-Verfahren zur Herstellung von Harnsäure, mit zwei nacheinanderfolgenden Zyklen, welches folgende Verfahrensschritte umfasst:

A) im ersten Zyklus (Hochdruckzyklus) enthält die von der Synthese herrührende Lösung hinter dem ersten isobaren Abscheider (E1) einen erheblichen Überschuss an $NH_3$ ($NH_3 : CO_2 \geqq 6$, in Mol ausgedrückt) und einen geringen $CO_2$-Anteil ($CO_2$:Harnsäure $\geqq$ 25 Gew.-%), ferner enthält sie vor dem zweiten isobaren Abscheider (E2) eine Menge an Ammoniumkarbamat, die neidriger ist, als der Karbamatgehalt der aus diesem Abscheider (E2) austretenden Lösung, wobei der Überschuss an somit erzeugtem Karbamat (in E2) zu einer Wärmeabgabe führt, die hinreichend ist, um einen erheblichen Anteil des vorliegenden $NH_3$ zu entfernen;

B) im zweiten Zyklus (Niederdruckzyklus) wird die aus E2 austretende Lösung auf 20 bis 30 Ata gebracht, und sie tritt in eine erste (Mitteldruck-)Säule (E3) ein, sie wird indirekt völlig erwärmt durch rückgeführten Niederdruckdampf aus der isobaren Kondensationsstufe (in E8) der aus dem Kopf von E2 austretenden Dampfs, wobei dieser rückgeführte Dampf einen Wäremgehalt besitzt, der hinreichend ist, um wenigstens das in E2 erzeugte überschüssige Karbamat zu zersetzen;

C) die aus E3 austretende Lösung wird auf etwa 5 Ata gebracht und tritt in eine zweite (Niederdruck)-Säule (E4) und sodann in einen Vakuumkonzentrierer (E5) ein, wo die Wärme der stark $NH_3$- und $CO_2$-haltigen, aus dem Kopf von E3 austretenden Dämpfe für den Betrieb von E4 und E5 ausgenutzt wird.

2. Verfahren nach Anspruch 1, mit folgenden Merkmalen:

— der Druck im "Hochdruck"-Zyklus beträgt 120 bis 250 kg/cm$^2$;

— die Temperatur der aus dem ersten isobaren Abscheider (E1) austretenden Lösung beträgt 150° bis 230°C;

— das genannte Molarverhältnis $NH_3 : CO_2$ ($\geqq$ 6 in Mol ausgedrückt, am Auslass des ersten Abscheiders) bezieht sich auf den gesamten $NH_3$- und $CO_2$-Gehalt, nicht nur dann, wenn eine chemische Bindung vorliegt, sondern auch auf die in Form von Harnsäure vorliegenden Mengen;

— der zweite Abscheider (E2) ist ein Fallschicht-Röhrenbündelwärmeaustauscher, in welchem die zu behandelnde Lösung einen $CO_2$-Gegenstrom trifft, der von aussen eingeleitet wird, wobei die Rohre vom kondensierten Dampf lediglich in ihrem oberen Abschnitt geheizt werden;

— der rückgeführte Dampf (von E8) steht unter einem Druck von ≧ 6,5 atm.abs;

— der Betriebsdruck im Behandlungsabschnitt des Vakuumkonzentrierers (E5) beträgt etwa 0,35 atm.abs;

— das Verhältnis zwischen eingeleitetem $CO_2$ und dem in den gleichen Abscheider eingelassenen Karbamat (CBE) ist sehr erheblich, und zwar:

$$(CO_2 \text{ eingeleitet}) : (CBE) > 2 \text{ (in Mol ausgedrückt).}$$

3. Verfahren nach Anspruch 1, bei welchem der erste isobare Abscheider (E1) des I.D.R.-Verfahrens ein vertikaler Wärmeaustauscher mit gebündelten Rohren vom "Fallschicht"- oder "Anstieg"-Type ist.

4. Verfahren nach Anspruch 1, bei welchem die Dampfspeisung (unter einem Druck von 18 bis 30 Ata) des zweiten isobaren Abscheiders (E2) auf null gebracht wird, wodurch eine rein adiabatsiche Trennung erzielt wird.

5. Verfahren nach Anspruch 1, bei welchem die aus E5 austretende Lösung in E6 unter Ausnutzung des rückgeführten Dampfs weiter konzentriert wird, wobei die derart erzielte konzentrierte Lösung in einen endgültigen Vakuumkonzentrierer (E7) eingeleitet wird, während die aus den Vakuumkonzentrierern (E5, E6 und E7) austretenden Dämpfe, die gewisse Mengen von $NH_3$ und $CO_2$ enthalten, in herkömmlicher Weise kondensiert werden, und wobei ein Rückgewinnungsturm (C3) die Rückgewinnung von zu kondensierenden Dampf ermöglicht (in E11), gleichzeitig mit den Dämpfen aus der Säule (E4), und wobei das derart erzielte Kondensat in den Destillierapparat (E4) (Gehäuseseite) zurückgeführt und die kondensierten Dämpfe aus E5 wieder komprimiert und in den Hochdruckzyklus zurückgeleitet werden.

6. Verfahren nach Anspruch 5, bei welchem (im zweiten Abscheider E2) das Verhältnis:

$$R = \frac{\text{vom kondensierten Dampf geheizter oberer Flächenbereich der Rohre}}{\text{Restlicher Teil der Rohroberfläche}}$$

R 1 beträgt und vorzugsweise zwischen ½ und ¼ liegt, wobei die obere Rohrfläche folglich ½ bis ⅕ (vorzugsweise ⅕ bis ⅓) der gesamten Oberfläche ausmacht.

7. Verfahren nach Anspruch 5, bei welchem die Verweilzeit der Harnsäurelösung im oberen Teil des austauschers (E2) 3 s oder weniger beträgt (vorzugsweise 2,5 und vorteilhafterweise 1,5 s), sodass das Verhältnis

$$\frac{\text{Verweilzeit im unteren Teil}}{\text{Verweilzeit im oberen Teil}}$$

gleich oder grösser 1 (vorzugweise gleich oder grösser als 2, vorzugsweise gleich oder grösser als 4) beträgt.

8. Verfahren nach Anspruch 5, mit folgenden Merkmalen:

a) die Harnsäuresynthese wird bei 150 bis 250°C unter einem Druck von 170 bis 220 atm durchgeführt;

b) die Temperatur der in den zweiten Abscheider (E2) eingeleiteten Lösung beträgt 190 bis 220°C;

c) die am Fuss dieses Abscheiders (E2) austretende Lösung besitzt eine Temperatur von 160 bis 190°C, vorzugsweise von 160 bis 175°C;

d) das als Extraktionsmittel in den gleichen Abscheider (E2) eingeleitete $CO_2$ besitzt eine Temperatur von 80 bis 140°C;

e) die aus dem ersten Abscheider (E1) austretende und in den zweiten Abscheider (E2) eingeleitete Lösung enthält wenig $CO_2$, derart, dass $CO_2$ : Harnsäure ≦ 22 Gew.-%;

f) das Molarverhältnis $NH_3$ : $CO_2$ der in den zweiten Abscheider eingleiteten Lösung ist gleich oder grösser als 10.

9. Verfahren nach Anspruch 5, bei welchem das Verhältnis zwischen der Austauschoberfläche ($S_{CO_2}$) des Abscheiders (E2) und der Austrauschoberfläche ($S_{NH_3}$) des Abscheiders E1 grösser als 1 ist, insbesondere: $S_{CO_2} : S_{NH_3} < 1$.

10. Verfahren nach Anspruch 5, bei welchem ein Teil der aus dem Kopf des Abscheiders (E2) austretenden Dämpfe direkt zum Synthesenreaktor zurückgeleitet werden, ohne den Hochdruckverdichter (E8) zu durchströmen, wobei diese Dämpfe an einer Stelle eingeleitet werden, die höher liegt, als der Einlass der vom ersten Abscheider (E1) kommenden Dämpfe.

11. Verfahren nach Anspruch 5, bei welchem die von den Vakuumkonzentrierern (E5 und E6) herrührenden Dämpfe (14) in einem wassergekühlten Apparat (E13) kondensiert werden, in welchen ebenfalls ein Teil der vom letzten Vakuumkonzentrierer (37) kommenden Dämpfe (15) (nach vorheriger Kompression in E12) eingeleitet werden, während der übrige Teil (25) als flüssige Phase in den Rückgewinnungsturm (C3) eingeleitet wird, wobei der Rückgewinnungsturm (C3) ein vertikales Fallschicht-Röhrenbündel aufweist, das durch aus dem Niederdruckdestillierapparat (E4) herrührende Dämpfe (11) beheitzt wird, und wobei die $NH_3$ enthaltenden, aus dem Turm (C3); austretenden Dämpfe (26) ebenfalls in dem wassergekühlten Apparat (E13) kondensiert werden, wonach das flüsige Kondensat (27), das aus dem Apparat (E13) austritt, in den Turm (C3) zurückgeführt wird, wobei das praktisch $NH_3$-freie Bodenprodukt

des Turmes (C3) zum Teil abgelassen (Leitung 30) und zum Teil zum Spülen der Synthesenventilierung (22) verwendet wird.

12. Kondensierapparat, insbesondere aur Anwendung im Rahmen des Apparats (E13) nach Anspruch 11, bestehend aus einem vertikalen Austauscher mit einem Röhrenbündel, in welchem das Kühlmittel das Röhrenbündel durchströmt, dadurch gekennzeichnet, dass die wenigstens teilweise zu kondensierenden Dämpfe in den untersten Teil des Gehäuses eintreten und aus dem Gehäuse an dessen Oberteil austreten, derart, dass sie im Gegenstrom in bezug auf das flüssige Kondensat strömen, wobei das Kondensat nach unten absinkt und aus dem unteren Gehäuseteil an einer Stelle austritt, die tiefer liegt, als der Dampfeinlass.

**Revendications**

1. Un procédé I.D.R. de préparation d'urée, en deux cycles sucessifs, présentant les caractéristiques suivantes:

A) dans le premier cycle (à haute pression), la solution provenant de la zone de synthèse renferme, en aval du premier séparateur (extracteur) isobare (E1) un fort excédent de $NH_3$ ($NH_3 = CO_2 \geqq 6$, exprimé en moles) et un faible pourcentage de $CO_2$ ($CO_2$ : Urée $\leqq$ 25% en poids) et elle renferme en amont du second séparateur isobare (E2) une quantité de carbamate d'ammonium inférieure à la quantité de carbamate contenue dans la solution sortant du même séparateur (E2), la production excédentaire de carbamate (dans E2) provoquant le dégagement d'une quantité de chaleur suffisante pour déplacer une proportion majeure de l'excédent de $NH_3$ qui y est contenu;

B) dans le second cycle (à basse pression), la solution sortant de E2 est portée à 20—30 ata et pénètre dans une première colonne (à pression moyenne) (E3) chauffée indirectement et complètement par de la vapeur récupérée provenant de la condensation isobare (dans E8) des vapeurs sortant de la tête de E2, ladite vapeur récupérée présentant un pouvoir calorifique tel qu'au moins ledit excédent de carbamate produit provenant de E2 soit décomposé;

C) la solution sortant de E3 est portée à sensiblement 5 ata et pénètre dans une seconde colonne (à basse pression) (E4) et ensuite dans un concentrateur à vide (E5), cependant que la chaleur des vapeurs quittant la tête de E3 et riches en $NH_3$ et $CO_2$ est utilisée pour assurer le fonctionnement de E4 et E5.

2. Un procédé selon la revendication 1, présentant les caractéristiques suivantes:

— la pression du cycle "à haute pression" est de 120 à 250 kg/cm$^2$;

— la température de la solution sortant du premier séparateur isobare (E1) est de 150 à 230°C;

— ledit rapport molaire $NH_3$ : $CO_2$ ($\geqq$ 6 à la sortie du premier séparateur, en moles), concerne les quantités totales de $NH_3$ et $CO_2$, non seulement à l'état chimiquement liés, mais également les quantités de ces composés qui sont présentes sous forme d'urée;

— ledit séparateur isobare (E2) est un échangeur de chaleur à écoulement descendant et à faisceau de tubes dans lequel la solution à séparer rencontre un contre-courant de $CO_2$ injecté à partir de l'extérieur, les tubes n'étant chauffés par de la vapeur en voie de condensation que dans leur partie supérieure;

— ladite vapeur de récupération (provenant de E8) présente une pression de $\geqq$ 6,5 atm.abs;

— la pression de service de la partie en fonctionnement dudit concentrateur à vide (E5) est sensiblement égale à 0,35 atm.abs.;

— le rapport $CO_2$ fraise introduit dans le second séparateur isobare ($CO_2$ inj.)/carbamate (CBE) pénétrant dans le même séparateur atteint une valeur très élevée, à savoir:

$$(CO_2 \text{ inj.}) : (CBE) > 2 \text{ (exprimé en moles).}$$

3. Un procédé selon la revendication 1, dans lequel ledit premier séparateur isobare (E1) du procédé I.D.R. est un échangeur de chaleur vertical comportant un faisceau de tubes, qui est choisi dand le groupe comprenant les échangeurs dits "à écoulement descendant" et les échangeurs dits "à écoulement ascendant".

4. Un procédé selon la revendication 1, dans lequel l'amenée de vapeur (à 18—30 ata) vers le second séparateur isobare (E2) est réduite à zéro, ce qui permet de réaliser une séparation (extraction) purement adiabatique.

5. Un procédé selon la revendication 1, dans lequel la solution quittant E5 est en outre concentrée dans E6 avec utilisation de la chaleur de la vapeur de récupération précitée, la solution concentrée ainsi obtenue étant transférée vers un concentrateur à vide final (E7), procédé dans lequel les vapeurs sortant des concentrateurs à vide (E5, E6 et E7) et renfermant certaines quantités de $NH_3$ et de $CO_2$ sont condensées d'une manière connue en soi, et dans lequel une colonne de récupération (C3) permet d'obtenir de la vapeur de récupération à condensée (dans E11), de même que des vapeurs provenant de ladite colonne (E4), les produits de condensation résultants étant recyclés vers ledit distillateur (E4) (côté enveloppe), cependant que les vapeurs condensées sortant de E5 sont comprimés à nouveau et recyclées dans le cycle à haute pression.

6. Un procédé selon la revendication 5, dans lequel (à l'intérieur dudit second séparateur E2) le rapport:

$$R = \frac{\text{Surface supérieure des tubes, chauffée par la vapeur condensée}}{\text{Partie restante de la surface des tubes}}$$

correspond à $R \leqq 1$, R étant compris, de préférence entre ½ et ¼, la partie supérieure de la surface des tubes représentant ainsi ½ à ¼ (de préférence ⅕ à ⅓) de la surface totale.

7. Un procédé selon la revendication 5, dans lequel le temps de séjour de la solution d'urée dans ladite partie supérieure de l'échangeur (E2) est égal ou inférieur à 3 s (de préférence 2,5, et avantageusement 1,5 à 2,5 s) le rapport

$$\frac{\text{Temps de séjour dans la partie inférieure}}{\text{Temps de séjour dans la partie supérieure}}$$

étant, par conséquent, égal où supérieur à 1 (de préférence égal ou supérieur à 2, et avantageusement égal ou supérieur à 4).

8. Un procédé selon la revendication 5, dans lequel:

a) la synthèse d'urée est effectuée à 150—200°C et sous 170—220 atm.;

b) la température de la solution introduite dans ledit second séparateur (E2) est de 190—220°C;

c) la température de la solution sortant de la cuve du même séparateur (E2) est de 150—190°C, de préférence 160—190°C et avantageusement 160—175°C;

d) la température du $CO_2$ injecté en tant qu'agent d'extraction dans ce même séparateur (E2) est de 80—140°C;

e) la solution sortant dudit premier séparateur (E1) et pénétrant dans ledit second séparateur (E2) renferme une faible quantité de $CO_2$, de façon telle que:

$$CO_2 : \text{urée} \leqq 22\% \text{ en poids;}$$

f) le rapport molaire $NH_3 : CO_2$ dans la solution pénétrant dans ledit second séparateur (E2) est égal ou supérieur à 10.

9. Un procédé selon la revendication 5, dans lequel le rapport de la surface d'échange ($S_{CO_2}$) dudit échangeur E2 à la surface d'échange ($S_{NH_3}$) dudit échangeur E1 est supérieur à 1, et dans lequel notamment: $S_{CO_2} : S_{NH_3} > 1$.

10. Un procédé selon la revendication 5, dans lequel une partie des vapeurs sortant de la tête dudit séparateur (E2) est recyclée directement vers le réacteur de synthèse, sans que ces vapeurs s'écoulent à travers le condenseur à haute pression (E8), ladite partie des vapeurs étant introduite en un point situé à un niveau plus élevé que l'entrée des vapeurs provenant dudit premier séparateur (E1).

11. Un procédé selon la revendication 5, dans lequel les vapeurs (14) provenant des concentrateurs à vide (E5 et E6) sont condensées dans un appareil refroidi à l'eau (E13) dans lequel on introduit également une partie des vapeurs (15) provenant du dernier concentrateur à vide (37) (après compression dans E12), la partie restante (25) étant amenée en phase liquide vers la tour de récupération (C3), procédé dans lequel, par ailleurs, ladite tour (C3) renferme un faisceau de tubes à écoulement descendant chauffé par les vapeurs (11) provenant dudit distillateur à basse pression (E4), et dans lequel les vapeurs renfermant du $NH_3$ et provenant de ladite tour (C3) sont également condensées dans ledit appareil refroidi à l'eau (E13), après quoi le condensat liquide sortant dudit appareil (E13) est recyclé vers ladite tour (C3) dont le produit de cuve, pratiquement exempt de $NH_3$, est en partie rejeté (conduite 30) et en partie utilisé (conduite 29) pour le rinçage des moyens de ventilation (22) de l'étape de synthèse.

12. Un appareil de condensation, se prêtant particulièrement à être utilisé pour la réalisation de l'appareil E13 selon la revendication 11, ledit appareil de condensation étant constitué par un échangeur de chaleur vertical renfermant un faisceau de tubes, ledit échangeur étant caractérisé en ce que les vapeurs à condenser (au moins partiellement) pénètrent dans la partie inférieure de l'enveloppe et quittent cette enveloppe par sa partie supérieure, de manière à s'écouler à contre-courant du condensat liquide qui descend et qui quitte la partie inférieure de ladite enveloppe en un point situé à un niveau plus bas que celui de l'entrée des vapeurs.

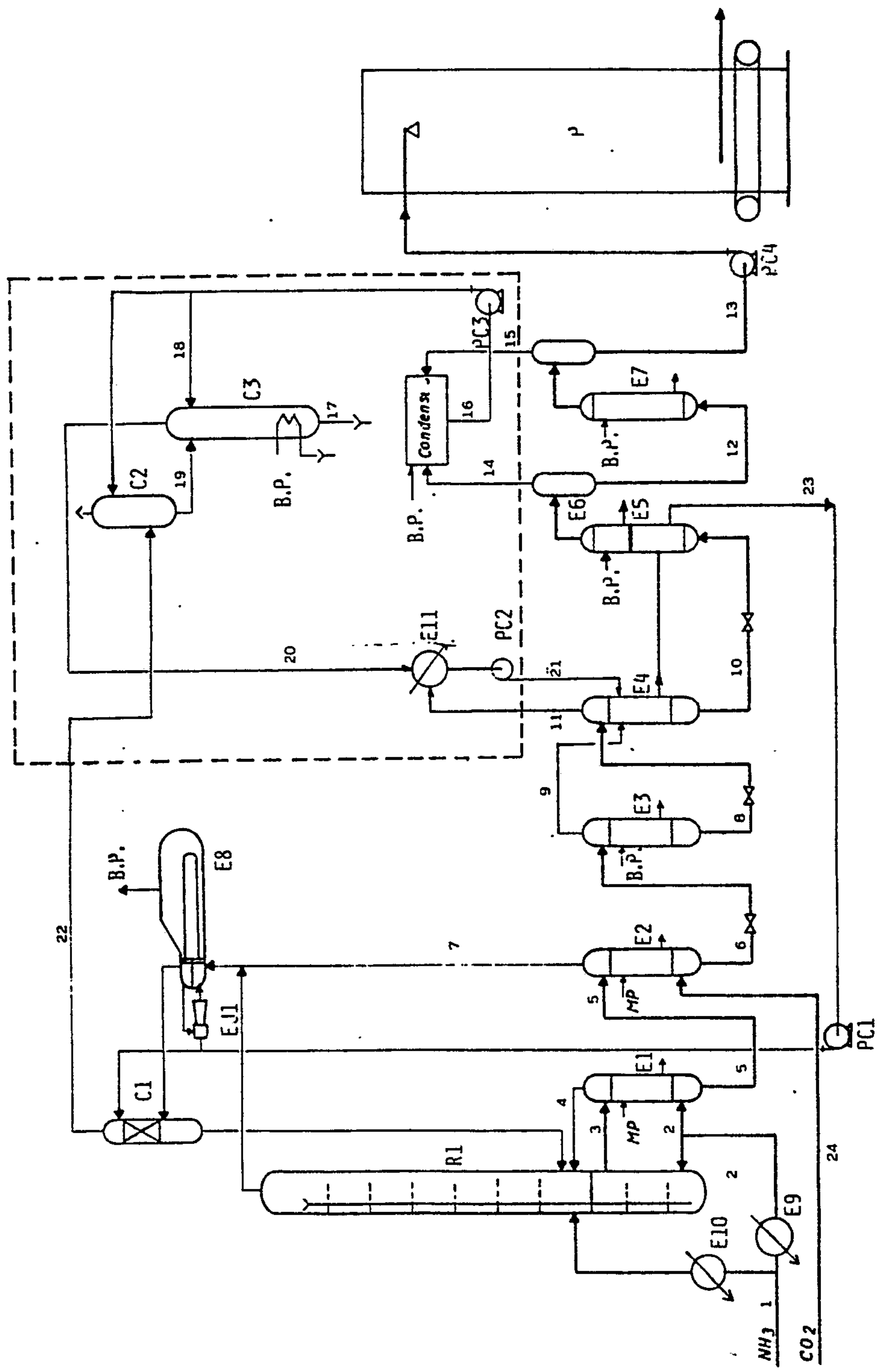
R1
E1
E2
E3
E4
E5
E6
E7
E8
E9
E10
E11
C1
C2
C3
EJ1
PC1
PC2
PC3
PC4
Condens
B.P.
MP
$NH_3$
$CO_2$

FIGURE 1

FIGURE 2

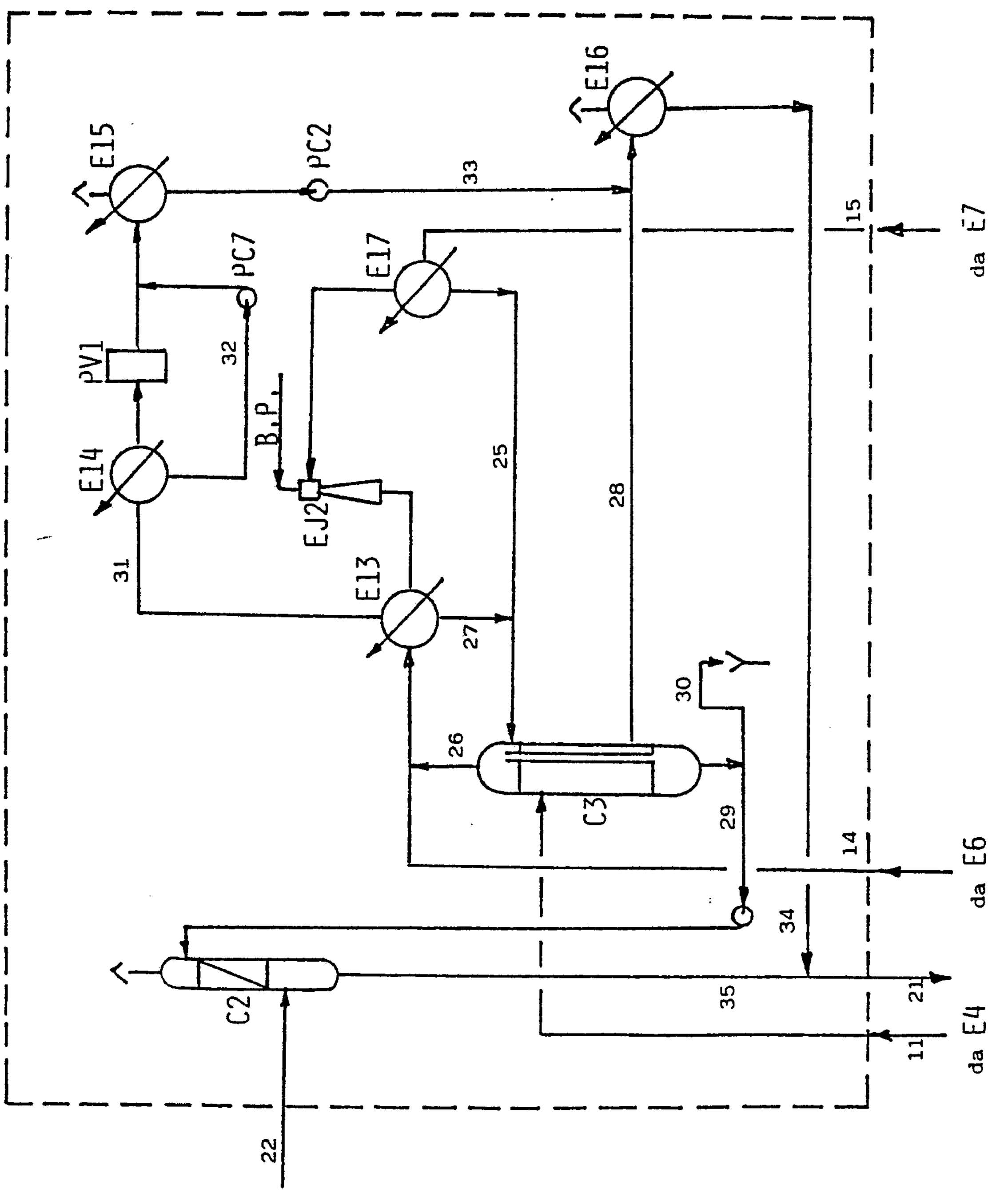
E15
PC2
33
E16
PC7
E17
15
da E7
PV1
32
B.P.
E14
25
28
EJ2
31
E13
27
30
26
C3
29
14
da E6
34
C2
35
21
11
da E4
22

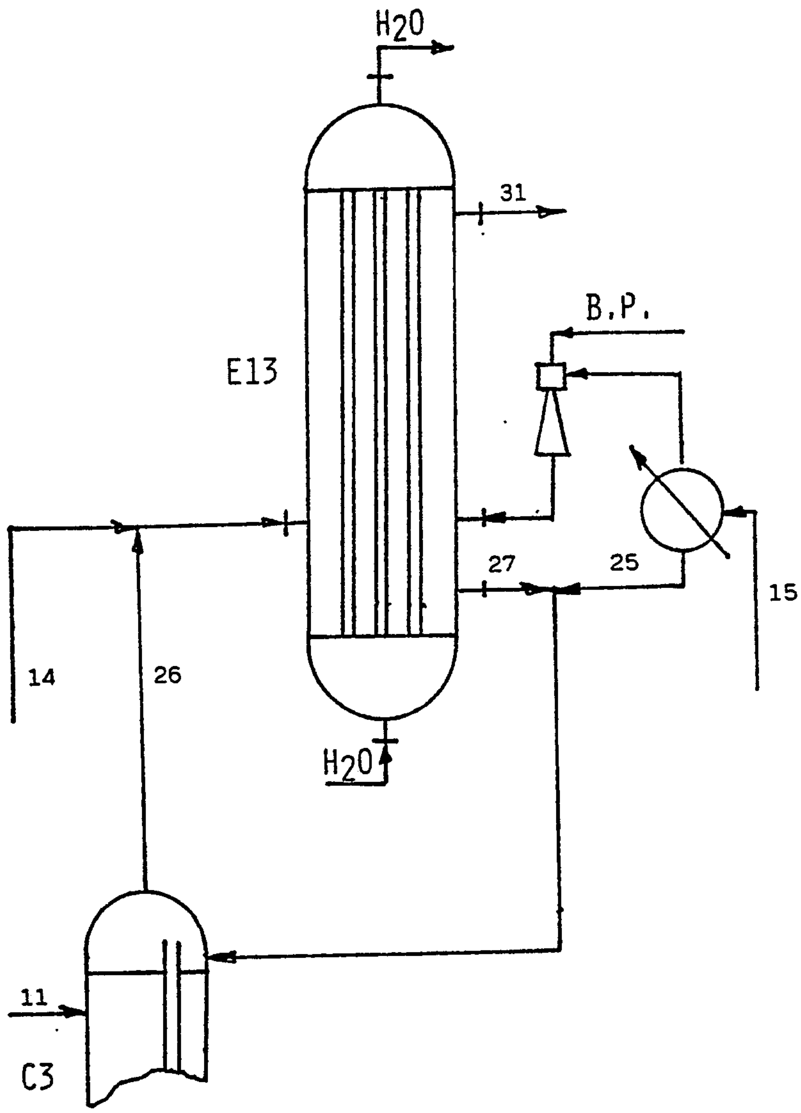


FIGURE 3

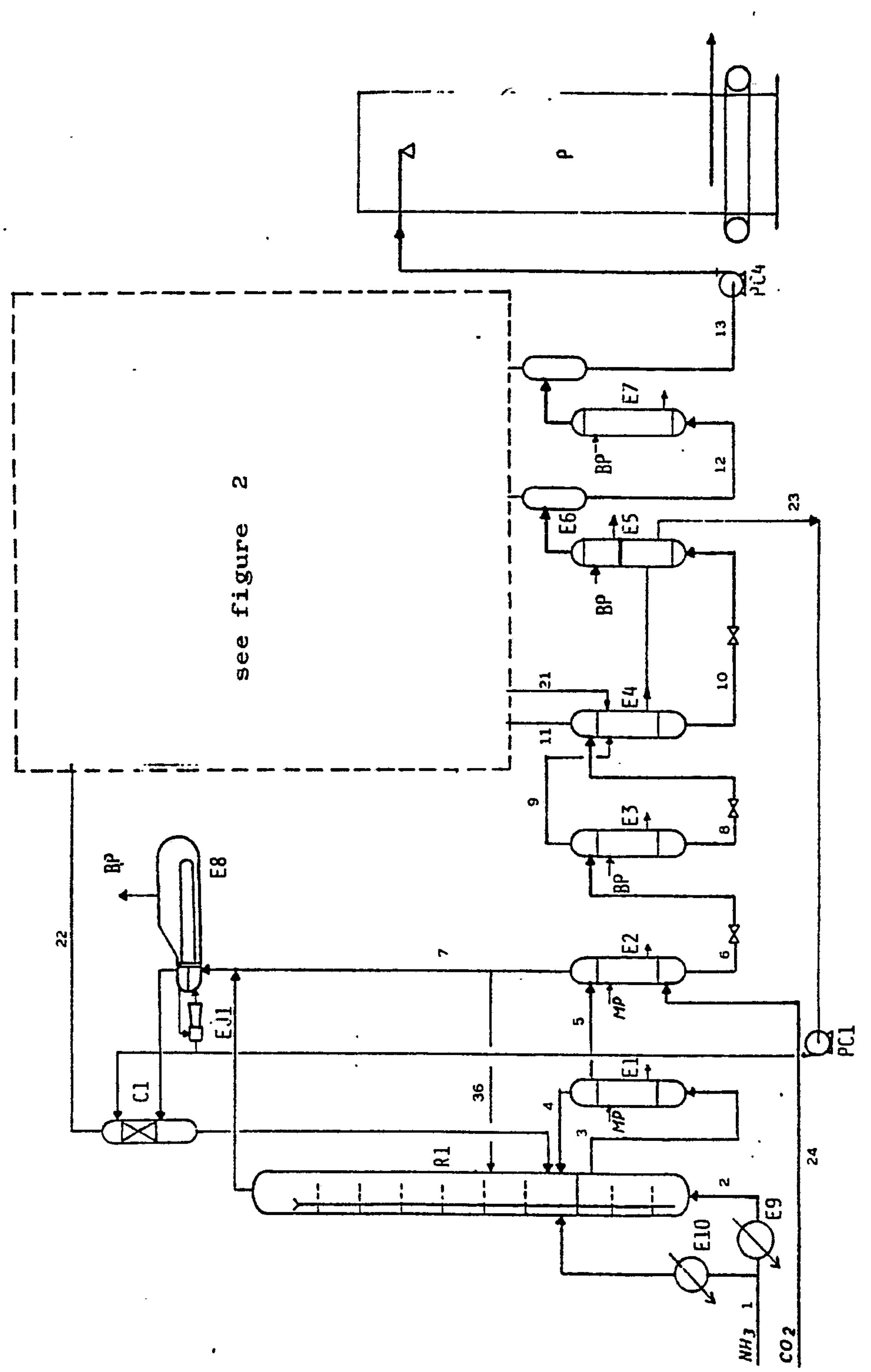
see figure 2
R1
C1
E1
E2
E3
E4
E5
E6
E7
E8
E9
E10
EJ1
PC1
PC4
BP
MP
P
$NH_3$
$CO_2$
1
2
3
4
5
6
7
8
9
10
11
12
13
21
22
23
24
36

FIGURE 4

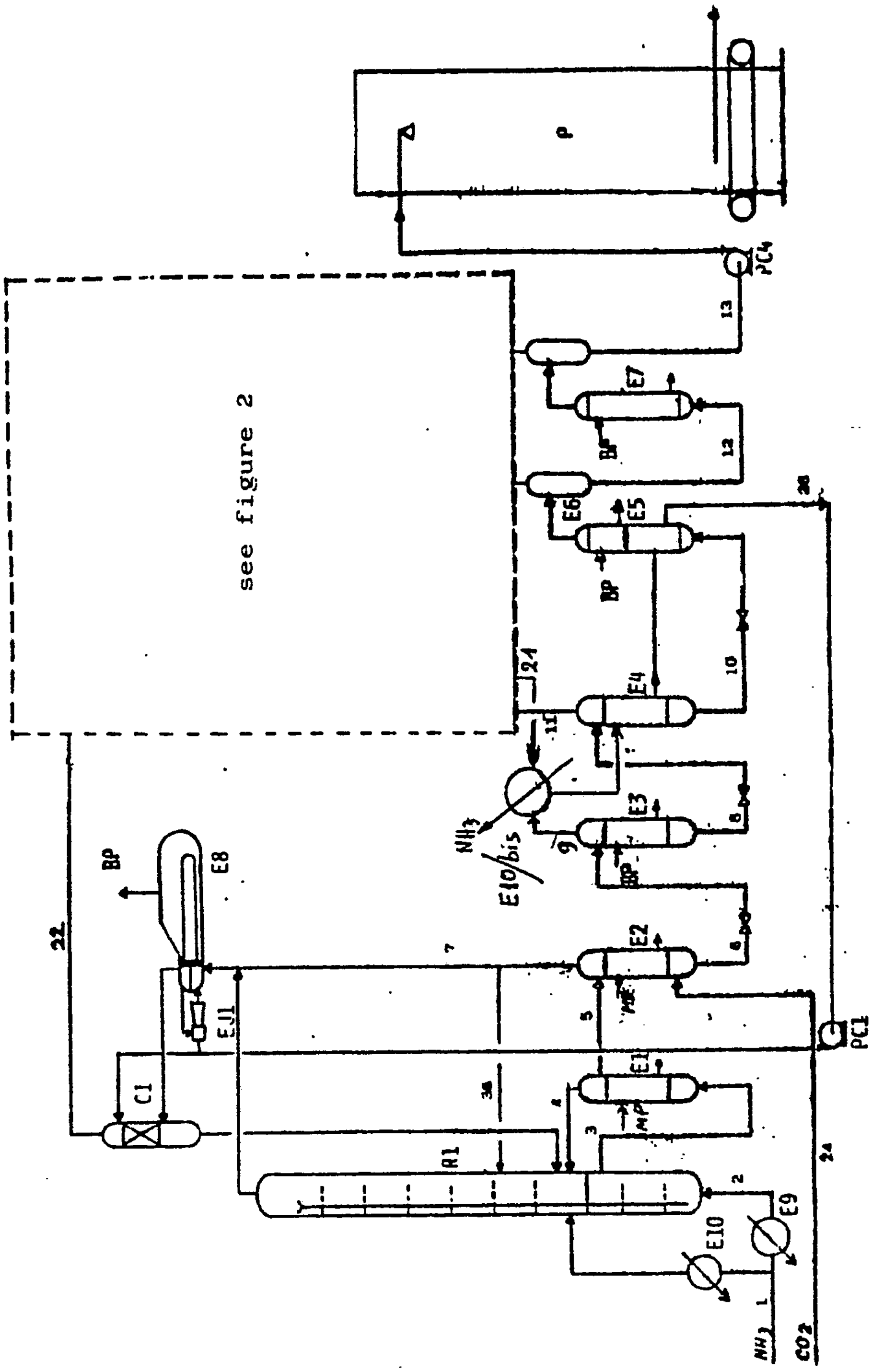
see figure 2
P
PC4
13
E7
12
BP
E6
E5
BP
24
E4
10
11
NH3
E10/bis
E3
9
8
BP
E2
6
7
5
22
BP
E8
EJ1
PC1
C1
E1
4
3
MP
R1
2
E9
E10
1
24
NH3
CO2

FIGURE 5